# EUROPÄISCHE PATENTSCHRIFT

(11) **EP 1 204 488 B1**
(45) Veröffentlichungstag und Bekanntmachung des Hinweises auf die Patenterteilung: **17.09.2008**
(21) Anmeldenummer: 01949318.8
(22) Anmeldetag: 08.05.2001
(51) Int. Cl.: B05D 3/06, C08F 291/18, G01N 33/543

(54) **VERFAHREN ZUR HERSTELLUNG VON ALS AUSGANGSPRODUKTE FÜR MIKROARRAYS DIENENDEN OBERFLÄCHENFUNKTIONALISIERTEN TRÄGERN ZUR IMMOBILISIERUNG VON BIOMOLEKÜLEN**
METHOD FOR PRODUCING SURFACE-FUNCTIONALIZED SUPPORTS USED AS STARTING PRODUCTS FOR MICROARRAYS, FOR IMMOBILIZING BIOMOLECULES
PROCEDE DE PRODUCTION DE SUPPORTS A SURFACE FONCTIONNALISEE SERVANT DE PRODUITS DE DEPART POUR JEUX ORDONNES DE MICRO-ECHANTILLONS, POUR L'IMMOBILISATION DE BIOMOLECULES

(30) Priorität: 02.06.2000 DE 10028851
(43) Veröffentlichungstag der Anmeldung: 15.05.2002
(73) Patentinhaber: EPPENDORF AG, 22339 Hamburg (DE)
(72) Erfinder: PLÜSTER, Wilhelm, 22397 Hamburg (DE); KÖHN, Heinz-Gerhard, 22339 Hamburg (DE); ULBRICHT, Mathias, 10407 Berlin (DE)
(74) Vertreter: Emmel, Thomas
(86) Internationale Anmeldenummer: PCT/EP2001/005173
(87) Internationale Veröffentlichungsnummer: WO 2001/094032

(56) Entgegenhaltungen:
- WO-A-00/12575
- WO-A-01/21326
- WO-A-97/31256
- WO-A-99/63385
- SCHULZ M ET AL: "FUNKTIONALISIERTE POLYMEROBERFLAECHEN" CLB. CHEMIE IN LABOR UND BIOTECHNIK, VERLAG BREIDENSTEIN, FRANKFURT AM MAIN, DE, Bd. 51, Nr. 3, 2000, Seiten 84-86, XP001015413 ISSN: 0943-6677
- REHMAN F ET AL: "Immobilization of acrylamide-modified oligonucleotides by co-polymerization" NUCLEIC ACIDS RESEARCH, OXFORD UNIVERSITY PRESS, SURREY, GB, Bd. 27, Nr. 2, 15. Februar 1999 (1999-02-15), Seiten 649-655, XP002155808 ISSN: 0305-1048

## Beschreibung

Die Erfindung bezieht sich auf ein Verfahren zur Herstellung von oberflächenfunktionalisierten Trägern zur Immobilisierung von Biomolekülen, die zur Herstellung von Mikroarrays verwendet werden sollen, auf ein Verfahren zur Herstellung solcher Mikroarrays ausgehend von den erfindungsgemäß hergestellten oberflächenfunktionalisierten Trägern sowie auf die mit den Verfahren herstellbaren Träger und Mikroarrays.

Unter Mikroarrays sollen im folgenden flächige Träger (Slides) verstanden werden, auf deren Oberfläche in definierten voneinander isolierten Bereichen (Spots) als Sonden (Sondenmoleküle) dienende Biomoleküle gekoppelt sind, die ihrerseits eine spezifische Bindung mit weiteren zu analysierenden Biomolekülen (Target-Molekülen) eingehen können. Diese Spots werden als zweidimensionale Objekte detektiert. Der Begriff Array ist weit zu fassen und schließt soweit er hier gebraucht wird auch andere Verteilungen von Spots auf der Trägeroberfläche ein, die nicht zwingend unter eine Arrayanordnung im strengen Sinne fallen.

Mit dem Begriff Träger werden im folgenden die nicht behandelten Glas- oder Kunststoffslides bezeichnet. Nach Funktionalisierung werden die Träger als funktionalisierte Träger bezeichnet, wobei hier noch keine Sondenmoleküle gekoppelt sind. Der Begriff Mikroarrays wird nur für funktionalisierte Träger verwendet, an denen Sondenmoleküle gekoppelt sind.

Mikroarrays werden insbesondere z.B. zur Analyse komplexer DNA- oder RNAhaltiger Gemische oder zur Proteinanalyse eingesetzt. Die mit Microarrays arbeitende Technik soll im folgenden auch als Array-Technik bezeichnet werden.

Als Sondenmoleküle für DNA- oder RNA-Analysen werden z.B. Oligonukleotide eingesetzt. Bei Proteinanalysen können Antigene oder Antikörper als Sondenmoleküle an die Träger gekoppelt werden. Insbesondere soll sich die Erfindung auf Verfahren zur Herstellung von Mikrochips für die. Nukleinsäureanalyse beziehen.

Die Sondenmoleküle können z.B. direkt auf die Oberfläche der Träger synthetisiert werden. Bevorzugt werden die Sondenmoleküle jedoch mittels einer als Spotting bezeichneten Technik auf die Trägeroberfläche abgesetzt, die dann so funktionalisiert sein muß, daß sie die Sondenmoleküle koppelt.

Zum Spotten, d.h. Absetzen von Tropfen mit gelösten Sondenmolekülen auf der Trägeroberfläche, werden z.B. Pin-Köpfe oder InkJet-Print-Verfahren eingesetzt, die automatisiert Sondenmoleküle enthaltende Spots bevorzugt in Arrayanordnung auf dem Träger erzeugen. In herkömmlichen Spotting-Verfahren betragen die Tropfengrößen üblicherweise zwischen 0,03 bis 2 nl. Diese Tropfen besitzen damit dreidimensionalen Charakter.

Die Immobilisierung der Sondenmoleküle mittels Spotting setzt eine entsprechende Funktionalisierung der Trägeroberfläche voraus. Bekannt ist in diesem Zusammenhang eine Beschichtung des Trägers mit Polylysin oder eine Silanisierung der Träger. Beide Verfahren haben Nachteile. So führt die Polylysin-Beschichtung zu unspezifischen Bindungen und einem ungewollten Verlaufen der Tropfen nach dem Spotten. Bei der Silanisierung von Glas-Slides kann es auch zu unspezifischen Signalen kommen, die die Interpretation der Ergebnisse erschweren.

Das US-Patent 5,858,653 beschreibt Träger für die Array-Technik, die an ihre Oberfläche gekoppelte hydrophile Polymere aufweisen, welche reaktive Gruppen enthalten, die unter Thermoaktivierung mit z.B. Oligonukleotiden eine kovalente Bindung eingehen können. Die Herstellung der Träger erfolgt durch Ankoppeln der zuvor synthetisierten Polymere an die Trägeroberfläche, ggf. unter Nutzung photoreaktiver Gruppen im Polymer. Nachteilig ist, daß sich die relativ großen Polymere nur mit geringer Belegungsdichte und in relativ ungleichmäßiger Verteilung an den Träger koppeln lassen.

Aufgabe der Erfindung ist es, Verfahren zur Herstellung von oberflächenfunktionalisierten Trägern und Mikroarrays für die Array-Technik zu schaffen, die die Nachteile des Standes der Technik überwinden.

Gelöst wird die Aufgabe mit Verfahren gemäß der Ansprüche 1 und 16.

Das erfindungsgemäße Verfahren gemäß Anspruch 1 betrifft die Herstellung von oberflächenfunktionalisierten Trägem, die dann gemäß Anspruch 16 zu Mikroarrays, insbesondere Mikrochips für die Nukleinsäureanalyse weitergebildet werden können.

Erfindungsgemäß ist nach Anspruch 1 zunächst vorgesehen, daß die Oberfläche eines Trägers zunächst mit einem Initiator beschichtet wird, wobei der Initiator an der Oberfläche des Trägers z.B. adsorbiert ggf. auch kovalent gebunden wird.

Dann wird die mit dem Initiator beschichtete Oberfläche in Kontakt mit einer mindestens eine erste Gruppe von polymerisierbaren Monomeren enthaltenden Lösung gebracht. Die Bedingungen unter denen die Monomere mit der aktivierten Trägeroberfläche in Kontakt gebracht werden sowie der zur Aktivierung eingesetzte Initiator sind so gewählt, daß die Monomere unter Vermittlung durch den Initiator an die Trägeroberfläche binden und davon ausgehend zu funktionellen Polymerketten polymerisieren.

Erfindungsgemäß ist weiterhin vorgesehen, daß die eingesetzten Monomere der ersten Gruppe Bindungsstellen enthalten, an die die als Sondenmoleküle eingesetzten Biomoleküle binden können, ggf. unter Ausbildung einer kovalenten Bindung.

Aus der WO 00/12575 sind polymere Festphasenträger bekannt, die nach einem ähnlichen Verfahren hergestellt werden und die insbesondere den speziellen Anforderungen der Spotting-Technologie genügen. Allerdings ist in der WO 00/12575 nicht angegeben, daß die dort beschriebenen funktionalisierten Träger zu Microarrays, insbesondere Mikrochips für die Nukleinsäureanalyse weiterverarbeitet werden können. Die in der WO 00/12575 beschriebenen Verwendungszwecke beschränken sich im wesentlichen auf die Herstellung von chemischen Substanzbibliotheken.

In einer erfindungsgemäßen Ausgestaltung kann vorgesehen sein, daß mindestens eine weitere zweite Gruppe von Monomeren mit der ersten Gruppe an den Träger copolymerisiert wird. Die Monomere der zweiten Gruppe tragen im wesentlichen keine Bindungsstellen für Biomoleküle und dienen z.B. zur Verteilung und Steuerung der Belegungsdichte des Trägers mit Bindungsstellen und zur Einstellung der Hydrophobie oder Hydrophilie der funktionellen Polymerketten.

Es kann weiterhin eine dritte Gruppe von Monomeren eingesetzt werden, die nicht kovalente Bindungsstellen aufweisen, die in der Lage sind, Sondenmoleküle zu den kovalenten Bindungsstellen zu dirigieren. Geeignet sind z.B. Monomere mit kationischen Gruppen wie z.B. die besonders für Nukleinsäuren geeignete Gruppen NR⁺₄ oder anionische bzw. chelatisierende Gruppen, die für Proteine geeignet sind.

Die Ausbildung der funktionellen Polymere kann entweder durch Polymerisation der Monomere der ersten Gruppe oder durch Copolymerisation der Monomere der ersten und zweiten, der ersten und dritten oder der ersten, zweiten und dritten Gruppe erfolgen.

Die Erfindung bietet eine ganze Reihe von Vorteilen. Ein wesentlicher Vorteil ist, daß die die Bindungsstellen tragenden funktionellen Polymerketten ausgehend von der Trägeroberfläche in situ gebildet werden. Die Trägeroberfläche reagiert also zunächst mit Monomeren, die aufgrund ihrer geringen Größe mit gut steuerbarer und auch falls gewünscht relativ hoher Dichte auf der Trägeroberfläche gekoppelt werden können. Die Trägeroberfläche kann also erfindungsgemäß deutlich dichter mit den in situ gebildeten funktionellen Polymerketten belegt werden, als dies bei der bekannten Kopplung von zuvor synthetisierten Polymeren an die Trägeroberfläche möglich ist.

Weiterhin ermöglicht das erfindungsgemäße Verfahren in besonders effektiver Weise eine dreidimensionale Verteilung der Bindungsstellen über der Trägeroberfläche. Dies führt zu einer erhöhten Bindungskapazität pro Fläche für Sondenmoleküle und zu einer Verringerung von unspezifischen Bindungen, woraus ein besseres Signal/Rausch-Verhältnis resultiert.

Ein weiterer Vorteil ist, daß die mit dem erfindungsgemäßen Verfahren in gut steuerbarer Dichte nebeneinander angeordneten funktionellen Polymerketten ein schnelles Verdampfen und ein Verlaufen von Flüssigkeit verhindern, was zu einer besseren Ausbeute bei der Bindung von Sondenmolekülen in einem Spot, einer verbesserten Gleichmäßigkeit über die Spotfläche und zu einer Verringerung von Kreuzkontaminationen zwischen benachbarten Spots führt. Durch die einstellbare dreidimensionale Struktur der funktionellen Polymerketten wird eine Stabilisierung der beim Spotten abgesetzten Tropfen gewährleistet. Die abgesetzten Tropfen behalten im Gegensatz zum Stand der Technik beim Absetzen auf die erfindungsgemäß hergestellten Träger im wesentlichen ihre dreidimensionale Struktur, was zu besser definierten Spots führt.

Als Träger können erfindungsgemäß Glas- oder Kunststoffträger (Slides) eingesetzt werden. Die Kunststoffträger können z.B. aus Polystyrol, Polycarbonat, Polyvinylclorid oder auch Polypropylen bestehen, um nur einige Beispiele zu nennen.

Die Träger besitzen in aller Regel eine flächige Form. In einer bevorzugten Ausgestaltung der Erfindung kann vorgesehen sein, daß in dem für die Funktionalisierung vorgesehenen Oberflächenbereich des Trägers Vertiefungen ausgebildet sind. Auf diese Weise kann in einfacher Weise eine Vergrößerung der funktionalisierbaren bzw. beladbaren Trägeroberfläche erreicht werden.

Die Vertiefungen können regelmäßig oder unregelmäßig in der Trägeroberfläche verteilt sein. Bevorzugt ist vorgesehen, daß Form und Größe der Vertiefungen übereinstimmen und daß weiterhin die Vertiefungen mit übereinstimmenden Abständen zueinander in der Trägeroberfläche ausgebildet sind. Es ergibt sich dann ein regelmäßiges Muster, das in vorteilhafter Weise reproduzierbar von den üblicherweise eingesetzten, ein Muster abfahrenden Spotting-Einrichtungen beladen werden kann.

Die Vertiefungen sind gemäß der Erfindung in Form einer mit ihrer Spitze in den Träger weisenden Pyramide oder Kegel ausgebildet. Insbesondere bei Vertiefungen mit dieser Form erhält man in optimaler Weise eine Vergrößerung der Trägeroberfläche. Es kann davon ausgegangen werden, daß eine kegelförmige Vertiefung eine um einen Faktor 2 bis 3 größere Oberfläche aufweist, als die Kegelgrundfläche (also eine vergleichbare Fläche bei einem planaren Träger), was im Endeffekt heißt, daß man den Träger mit höherer Dichte funktionalisieren und dementsprechend mit mehr Sondenmolekülen beladen kann.

In Abhängigkeit von der gewählten Art der Beschichtung mit Initiatoren kann es erforderlich sein, daß die Träger in einem Vorschritt noch zusätzlich behandelt werden, um z.B. überhaupt eine Kopplung des Initiators an ihrer Oberfläche zu ermöglichen. Eine gängige Methode für Träger aus Glas ist z.B. die Silanisierung. Auch dies gehört zum Stand der Technik. Es wird darum an dieser Stelle nicht weiter darauf eingegangen. Bei Trägern aus Kunststoff ist in aller Regel eine solche Behandlung in einem Vorschritt nicht erforderlich.

Als Initiatoren eignen sich z.B. thermisch-, photochemisch- oder redoxaktivierbare Verbindungen wie z.B. Benzoinderivate, Azoverbindungen oder Peroxide. Ein besonders geeigneter Initiator ist z.B. Benzophenon. Bei Benzophenon handelt es sich um einen mittels UV-Licht aktivierbaren Initiator. Zur Beschichtung des Trägers mit Benzophenon reicht es aus, daß der Träger für eine bestimmte Dauer in eine benzophenonhaltige Lösung getaucht wird.

In einem nächsten Schritt wird der z.B. mit Benzophenon beschichtete Träger mit den Monomeren equilibriert. Dann erfolgt im Falle der Benzophenon-Aktivierung eine Bestrahlung mit UV-Licht, wobei das Benzophenon eine Bindung der Monomere an die Trägeroberfläche vermittelt.

Die beschriebene In-Situ-Technik unter Vermittlung eines UV-induzierbaren Initiators wird als Photo-Initiierte-Pfropf-Polymerisation bezeichnet und ist z.B. in der Veröffentlichung von "Ulbricht et al." in "Colloids and Surfaces Vol 138, 1998, S. 353 beschrieben. Die dort beschriebenen mittels Propf-Polymerisation herstellbaren Substrate sollen insbesondere zu Ultrafiltrationszwecken eingesetzt werden. Ein Einsatz in der Array-Technik sowie die gezielte Herstellung für diese Technik ist der Veröffentlichung nicht entnehmbar.

Denkbar sind selbstverständlich auch andere Initiatoren, die aufgrund ihrer speziellen Konfiguration oder Reaktivität eine Bindung zwischen der Trägeroberfläche und dem Monomer vermitteln können. Denkbar ist dabei, daß der Initiator auch in das gebildete Molekül eingebaut wird oder aber auch wie z.B. im Falle des Benzophenon, daß der Initiator nur die Bindung vermittelt und nicht selbst eingebaut wird.

Als Monomere eignen sich alle Verbindungen, die gut polymerisierbar sind wie z.B. Acrylsäure, Methacrylsäure, Derivate der Acrylsäure oder Methacrylsäure und Vinyl- und Allylverbindungen.

Besonders geeignete Monomere der ersten Gruppe, die zusätzlich Bindungsstellen aufweisen, die in der Lage sind, die gewünschten Sondenmoleküle ggf. kovalent zu binden, sind z.B. Acrylsäure, Glycidylmethacrylat oder Aminoalkylmethacrylat.

Als Monomere der zweiten Gruppe eignen sich alle Verbindungen, die Eigenschaften besitzen, um die Hydrophilie oder Hydrophobie der funktionellen Polymerketten einzustellen. Besonders geeignet sind z.B. Polyäthylenglykol, Methacrylate oder Hydroxymethylmethacrylamid.

Die Bindungsstellen sind in der Regel funktionelle Gruppen, die in den Monomeren enthalten sind. Insbesondere sind solche Bindungsstellen COOH-, SH-, NH₂-, Epoxid-, oder Thiolgruppen, die eine kovalente Bindung des Sondenmoleküls ermöglichen. Die Auswahl der Monomere bzw. der als Bindungsstellen dienenden funktionellen Gruppen kann in einfacher Weise an die zu bindenden Sondenmoleküle angepaßt erfolgen. Für den Fachmann stellt dies kein Problem dar.

Die erfindungsgemäß oberflächenfunktionalisierten Träger sollen hauptsächlich nach Kopplung mit Sondenmolekülen in definierten Spots als Mikroarrays Anwendung finden. Die Array-Technik ist insbesondere in Verbindung mit Nukleinsäure-Analysen eine gängige Methode. Es soll darum an dieser Stelle nicht näher darauf eingegangen werden.

Wesentliches Merkmal dieser Technik ist, daß Mikroarrays eingesetzt werden, an denen in definierten Orten Sondenmoleküle gekoppelt sind, die hochspezifische Bindungseigenschaften für z.B. Nukleinsäuremoleküle mit bestimmten Basensequenzen oder Proteine mit bestimmten immunologischen Eigenschaften haben.

Die Belegung der definierten Orte mit solchen Sondenmolekülen erfolgt in der Regel wie erwähnt automatisiert mittels einer als Spotting bezeichneten Technik.
Spotting ist eine bekannte Technik, auf die an dieser Stelle nicht weiter eingegangen werden soll.

Die erfindungsgemäß hergestellten oberflächenfunktionalisierten Träger können nun z.B. mittels Spotting-Techniken mit Sondenmolekülen belegt werden, und auf diese Weise zu Mikroarrays weiterverarbeitet werden.

Es lassen sich dabei z.B. Plasmid-DNA, Cosmid-DNA, Bakteriophagen-DNA, genomische DNA, RNA, cDNA, pDNA und Oligo-Nukleotide koppeln, um nur einige Beispiele zu geben. Denkbar ist selbstverständlich auch, daß an die genannten Bindungsstellen Antigene bzw. Antikörper zur Proteinanalyse gekoppelt werden oder andere Biomoleküle.

Die Erfindung ist nicht nur auf Verfahren zur Herstellung von oberflächenfunktionalisierten Trägern oder Mikroarrays beschränkt. Sie deckt vielmehr auch nach diesem Verfahren herstellbare funktionalisierte Träger und Arrays ab.

Insbesondere soll die Erfindung Verfahren zur Herstellung von oberflächenfunktionalisierten Trägern abdecken, die zu Mikro-Chips für Nukleinsäureanalyse weiterverarbeitet werden können und entsprechende Mikrochips.

Im folgenden soll die Erfindung anhand eines Beispieles und dreier Figuren näher erläutert werden.
- Figur 1: dokumentiert das Bindungsverhalten eines Ausführungsbeispieles des erfindungsgemäß herstellbaren funktionalisierten Trägers,
- Figur 2: zeigt eine Ausführung eines erfindungsgemäß einsetzbaren Trägers in Aufsicht,
- Figur 3: zeigt einen ausgehend von dem Träger aus Figur 2 hergestellten oberflächenfunktionalisierten Träger im Schnitt.

### Beispiel:

### Herstellung von funktionalisierten Glasträgern

Es wurden einseitig silanisierte Glasträger (CCT, Jena) eingesetzt. Die Glasträger wurden für 15 min. in eine 100 mM Lösung von Benzophenon in Aceton eingetaucht, dann mit einer mM-Lösung von Benzophenon in Aceton gespült und anschließend an der Luft getrocknet. Danach wurden die Glasträger mit der silanisierten und der mit Benzophenon beschichteten Seite auf die unten angegebenen Monomerlösungen unter Zusatz von 1 mM Natriumperiodat ohne Einschluß von Luftblasen zwischen Glas und Lösung aufgebracht. Nach 15 min. Equilibrierzeit wurden die auf der Monomerlösung schwimmenden Proben durch das Glas hindurch belichtet (t_{UV}). Nach weiteren 15 min. Nachreaktionszeit wurden die Träger extensiv mit Wasser, dann mit Aceton und anschließend wieder mit Wasser gewaschen. Anschließend wurden die Proben getrocknet.
A) Als Monomer-Lösung wurde Acrylsäure in einer Konzentration 25g/l Wasser verwendet. Diese Monomer-Lösung wurde bei unterschiedlichen Belichtungszeiten (t_{UV}) behandelt.
Die durch diese Monomere bereitgestellten Bindungsstellen waren Carboxylgruppen.
Die unterschiedlichen Varianten sind im folgenden zusammengefaßt:

| | | |
|---|---|---|
| 1. | Acrylsäure 25g/l | 7,5 min t_{UV} |
| 2. | Acrylsäure 25g/l | 10 min t_{UV} |
| 3. | Acrylsäure 25g/l | 15 min t_{UV} |

**B)** Als Monomer-Lösung wurde Glycidylmethacrylat in einer Konzentration von 10g/l Wasser und 20 g/l Wasser mit jeweils 25g/l Wasser Hydroxymethylmethacrylamid eingesetzt. Auch hier wurden unterschiedliche Belichtungszeiten von 10 min (10 bzw 20 g/l Wasser) bzw. 15 min t_{UV} (nur bei 20 g/l Wasser) eingesetzt.

Die durch diese Monomere bereitgestellten Bindungsstellen waren EpoxidGruppen.

Die unterschiedlichen Varianten sind im folgenden zusammengefaßt:

| | | |
|---|---|---|
| 1. | Glycidylmethacrylat 10g/l | 10 min t_{UV} |
| 2. | Glycidylmethaclylat 20g/l | 10 min t_{UV} |
| 3. | Glycidylmethacrylat 20g/l | 15 min t_{UV} |
| + jeweils 25g/l Hydroxymethylmethacrylamid | | |

Zur Probe wurden eine Biomolekül-Immobilisierung und -Assay durchgeführt.
**A)** Biotin wurde via N-Aminoethylbiotinamid ("Biotinamin"; Molecular Probes) an die mit EDC/NHS für 1h bei 25C° aktivierten Oberflächen gekoppelt (Reaktion bei pH= 7,4 für 5 h bei 25C°); Blank-Probe wurden analog, aber ohne "Biotinamin" präpariert.
**B)** Biotin wurde via "Biotinamin" an die Oberflächen gekoppelt (Reaktion bei pH= 9,6 über Nacht bei 25 C°). Blank-Proben wurden durch Inkubation in Puffer bei pH= 9,6 über Nacht bei 25C° erhalten.

Der Biotin-Assay wurde für A und B mit jeweils 16 mm² funktionalisiertem Glas durchgeführt: 1. Inkubation mit einem Streptavidin-Alkalischen Phosphatase-Konjugat für 30 min bei Raumtemperatur (RT); 2. Reaktion mit Paranitrophenylphosphat (PNP) für 30 min bei 37C° und photometrische Messung des Umsatzes bei 405 nm.

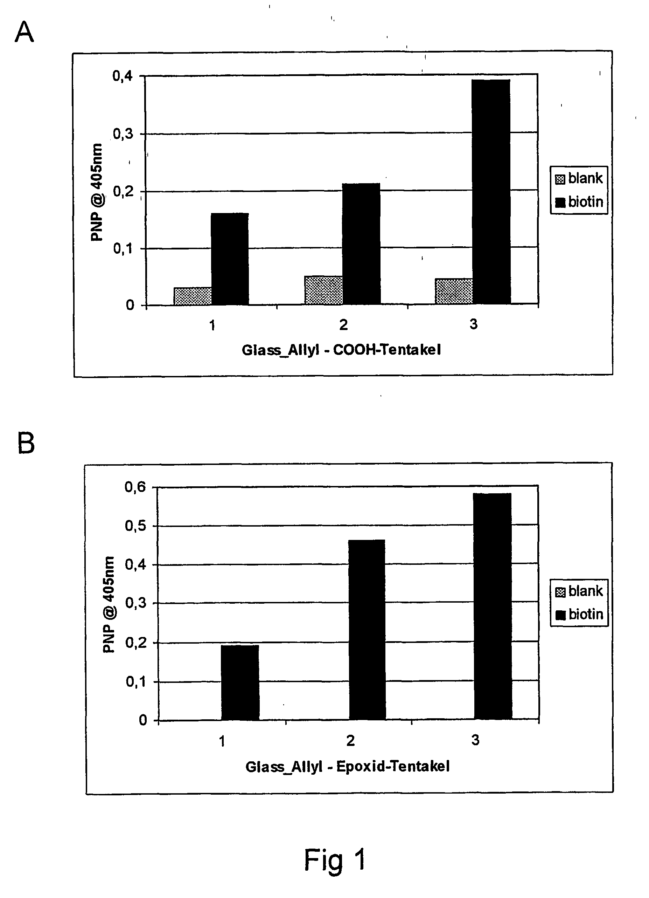

Die Ergebnisse der photometrischen Messung zeigt die Fig. 1.

Man erkennt für beide Fälle eine sehr effiziente Bindung von Biotin an die funktionalisierten Träger. Das Hintergrundsignal (hier durch unspezifische Bindung des Streptavidin-Konjugates) kann dabei durch entsprechende Auswahl der Monomere (B) bzw. über die Steuerung der Belegungsdichte (A 2 und 3) minimiert werden.

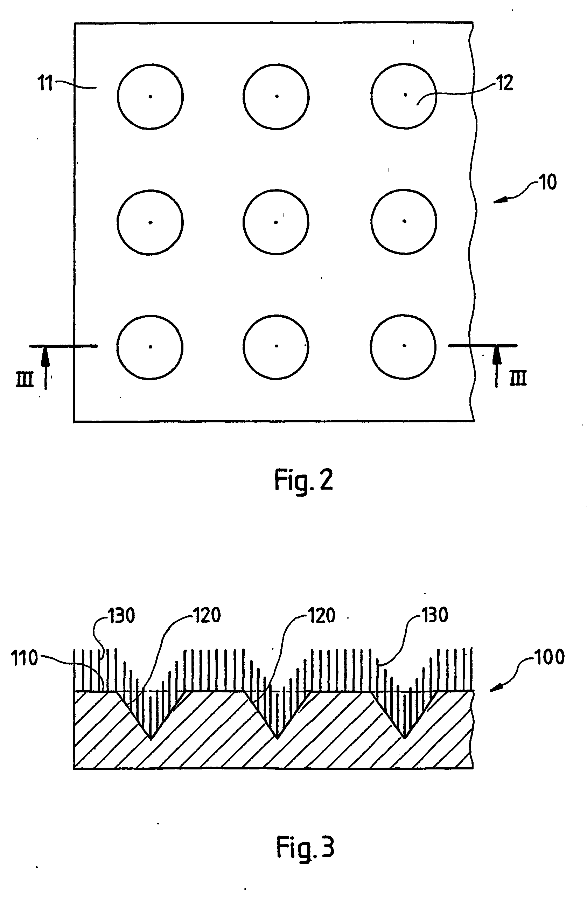

Figur 2 zeigt einen flächigen Träger 10 mit einem zur Funktionalisierung vorgesehenen Oberflächenbereich 11, in dem in regelmäßigen Abständen kegelförmige Vertiefungen 12 vorgesehen sind. Die kegelförmigen Vertiefungen können z.B. einen Durchmesser von ca. 20 µm haben und weisen zueinander einen Abstand von ebenfalls 20 µm auf. Übliche Spots haben einen Durchmesser von ca. 100 bis 150 µm und decken damit zwischen 20 bis 40 kegelförmige Vertiefungen auf der Oberfläche 11 des Trägers 10 ab. Diese Angaben sind natürlich nicht zwingend. Es ist genauso gut möglich, Träger einzusetzen, in deren Oberfläche Vertiefungen mit anderer Form, Durchmesser etc. ausgebildet sind. Wesentlich ist jedoch, daß die Vertiefungen sich möglichst in den Träger hinein verjüngen, da die dadurch entstehenden Schrägflächen die gewünschte Oberflächenvergrößerung bewirken.

Der in Figur 2 dargestellte Träger 10 ist noch nicht oberflächenfunktionalisiert. Den oberflächenfunktionalisierten Zustand zeigt Figur 3 in einer Schnittdarstellung. Man erkennt wiederum einen Träger 100 mit funktionalisierter Oberfläche 110, in der Vertiefungen 120 ausgebildet sind. Die im Rahmen der Funktionalisierung kovalent an die Oberfläche 110 gekoppelten Polymerketten sind mit 130 bezeichnet. Es wird deutlich, daß pro kegelförmige Vertiefung 120 mehr Polymerketten 130 gekoppelt werden können, als dies auf einem vergleichbaren planaren Abschnitt der Oberfläche 110 möglich wäre, die größenmäßig der Kegelgrundfläche entspricht.

## Patentansprüche

1. Verfahren zur Herstellung von als Ausgangsprodukte für die Herstellung von Mikroarrays dienenden oberflächenfunktionalisierten Trägern für die Immobilisierung von Biomolekülen, bei dem die Oberfläche eines Trägers mit einem Initiator beschichtet und die beschichtete Oberfläche dann in Kontakt mit einer mindestens eine erste Gruppe von polymerisierbaren Monomeren enthaltenen Lösung gebracht wird, wobei die Monomere Bindungsstellen enthalten, an die Biomoleküle (Sondenmoleküle) binden können und die Bedingungen, unter denen die Monomer-Lösung in Kontakt mit dem aktivierten Träger gebracht wird, so gewählt sind, daß die Monomere unter Vermittlung durch den Initiator an den Träger binden und davon ausgehend zu funktionellen Polymerketten polymerisieren dergestalt, daß eine an der Oberfläche des Trägers fixierte Struktur von benachbarten funktionellen Polymerketten entsteht, wobei die Träger flächig ausgebildet sind und in einem für die Funktionalisierung vorgesehenen Oberflächenbereich Vertiefungen in Form einer mit ihrer Spitze in den Träger weisenden Pyramide oder eines Kegels aufweisen.

2. Verfahren nach Anspruch 1, **dadurch gekennzeichnet, daß** als Initiator lichtaktivierbare Substanzen, insbesondere Benzophenon, eingesetzt werden.

3. Verfahren nach Anspruch 1 oder 2, **dadurch gekennzeichnet, daß** die Träger aus Glas oder Kunststoff bestehen.

4. Verfahren nach Anspruch 3, **dadurch gekennzeichnet, daß** die eingesetzten Kunststoffträger aus Polystyrol, Polycarbonat, Polyvenylclorid, oder Polypropylen bestehen.

5. Verfahren nach Anspruch 4, **dadurch gekennzeichnet, daß** bezüglich Form und Größe übereinstimmende Vertiefungen mit übereinstimmenden Abständen zueinander in der Trägeroberfläche vorgesehen sind.

6. Verfahren nach einem der Ansprüche 1 bis 5, **dadurch gekennzeichnet, daß** die im Verfahren eingesetzten Träger aus Glas in einem ersten Schritt silanisiert werden.

7. Verfahren nach einem der Ansprüche 1 bis 6, **dadurch gekennzeichnet, daß** mindestens eine weitere zweite Gruppe von Monomeren mit der ersten Gruppe an den Träger copolymerisiert wird, wobei die Monomere der zweiten Gruppe im wesentlichen keine Bindungsstellen für Biomoleküle aufweisen.

8. Verfahren nach Anspruch 7, **dadurch gekennzeichnet, daß** die Auswahl der Monomere der zweiten Gruppe und die Bestimmung der einzusetzenden Monomerkonzentration gezielt zur Einstellung einer gewünschten Hydrophilie bzw. Hydrophobie der copolymerisierten funktionellen Polymerketten erfolgt.

9. Verfahren nach Anspruch 8, **dadurch gekennzeichnet, daß** die Auswahl und eingesetzte Konzentration der Monomere der zweiten Gruppe zu copolymerisierten funktionellen Polymerketten führt, die einen beim Spotten abgesetzten Tropfen in seiner dreidimensionalen Struktur aufnehmen und halten.

10. Verfahren nach einem der Ansprüche 1 bis 9, **dadurch gekennzeichnet, daß** die Monomere der ersten und zweiten Gruppe aus folgenden Verbindungen ausgewählt sind: Acrylsäuren, Methacrylsäuren, Derivate der Acrylsäure oder Methacrylsäure, und Vinyl- oder Allylverbindungen.

11. Verfahren nach Anspruch 10, **dadurch gekennzeichnet, daß** die Monomere der ersten Gruppe aus folgenden Verbindungen ausgewählt sind: Acrylsäuren, Glycidylmethacrylate oder Aminoalkylmethacrylate.

12. Verfahren nach Anspruch 11, **dadurch gekennzeichnet, daß** die Monomere der zweiten Gruppe aus folgenden Verbindungen ausgewählt sind: Polyäthylenglykole, Methacrylate oder Hydroxymethylmethacrylamide.

13. Verfahren nach einem der Ansprüche 1 bis 12, **dadurch gekennzeichnet, daß** die in den Monomeren der ersten Gruppe enthaltenen Bindungsstellen funktionelle Gruppen sind, insbesondere COOH-, SH-, NH₂-, Epoxid-, oder Thiolgruppen.

14. Verfahren zur Herstellung von Mikroarrays, bei dem in definierten Bereichen auf einem gemäß der Ansprüche 1 bis 13 hergestellten oberflächenmodifizierte Träger in Lösungsmittel gelöste Sondenmoleküle tropfenweise abgesetzt werden.

15. Verfahren zur Herstellung von Mikroarrays nach Anspruch 14, bei dem die oberflächenmodifizierten Träger mittels Spotting mit den Sondenmolekülen belegt werden.

16. Verfahren nach Anspruch 14 oder 15, **dadurch gekennzeichnet, daß** als Sondenmoleküle Oligonukleotide oder Antikörper eingesetzt werden.

17. Verfahren zur Herstellung von Mikroarrays nach einem der Ansprüche 14 bis 16, **dadurch gekennzeichnet, daß** die Mikroarrays als Mikrochips für die Nukleinsäureanalyse ausgebildet werden.

18. Oberflächenmodifizierter Träger herstellbar gemäß der Ansprüche 1 bis 13.

19. Träger für die Array-Bindungstechnik herstellbar gemäß der Ansprüche 14 bis 17.

## Claims

1. A process for the production of surface-functionalized supports that serve as starting products for the production of microarrays for immobilizing biomolecules, in which the surface of a support is coated with an initiator and the coated surface is then put in contact with a solution containing at least one first group of polymerizable monomers, with the monomers containing binding sites to which biomolecules (probe molecules) can bind, and the conditions under which the monomer-solution is put in contact with the activated support being selected such that the monomers, mediated by the initiator, bind to the support and starting therefrom polymerize to functional polymer chains in such a manner that a structure fixed on the surface of the support is formed consisting of adjacent functional polymer chains, wherein the supports have a planar configuration and are provided with depressions in a surface area intended for functionalization, the depressions having the shape of pyramids or cones whose tips point toward the support.

2. The process according to claim 1, **characterised in that** substances that can be activated by light, especially benzophenone, are used as the initiator.

3. The process according to claim 1 or 2, **characterised in that** the supports are made of glass or plastic.

4. The process according to claim 3, **characterised in that** the plastic supports used consist of polystyrene, polycarbonate, polyvinyl chloride or polypropylene.

5. The process according to claim 4, **characterised in that** the depressions whose shapes and sizes match each other are positioned equidistantly from each other in the surface of the support.

6. The process according to one of the claims 1 to 5, **characterised in that** the supports made of glass used in the process are silanized in a first step.

7. The process according to one of the claims 1 to 6, **characterised in that** at least an additional second group of monomers is copolymerized with the first group on the support, whereby the monomers of the second group essentially do not have any binding sites for biomolecules.

8. The process according to claim 7, **characterised in that** the selection of the monomers of the second group and the determination of the monomer concentration to be used are both carried out with an eye towards the adjustment of the desired hydrophilia or hydrophobia of the copolymerized functional polymer chains.

9. The process according to claim 8, **characterised in that** the selection of the monomers of the second group and the concentration used lead to copolymerized functional polymer chains that receive and retain the three-dimensional structure of a drop deposited during spotting.

10. The process according to one of the claims 1 to 9, **characterised in that** the monomers of the first and second group are selected from among the following compounds: acrylic acids, methacrylic acids, derivatives of acrylic acid or methacrylic acid and vinyl or allyl compounds.

11. The process according to claim 10, **characterised in that** the monomers of the first group are selected from among the following compounds: acrylic acids, glycidyl methacrylates or aminoalkyl methacrylates.

12. The process according to claim 11, **characterised in that** the monomers of the second group are selected from among the following compounds: polyethylene glycols, methacrylates or hydroxymethyl methacrylamides.

13. The process according one of the claims 1 to 12, **characterised in that** the binding sites contained in the monomers of the first group are functional groups, particulary COOH-, SH-, NH₂-, expoxide- or thiol-groups.

14. A process for the production of microarrays, wherein probe molecules dissolved in a solvent are deposited dropwise in defined areas on a surface-modified support produced according to claims 1 to 13.

15. The process for the production of microarrays according to claim 14, wherein the surface-modified supports are loaded with probe molecules by means of spotting.

16. The process according to claim 14 or 15, **characterised in that** oligonucleotides or antibodies are employed as probe molecules.

17. The process for the production of microarrays according to one of the claims 14 to 16, **characterised in that** the microarrays are made as microchips for nucleic acid analysis.

18. A surface-modified support than can be produced according to claims 1 to 13.

19. A support for the array binding technique that can be produced according to claim 14 to 17.

## Revendications

1. Procédé de fabrication de supports à surface fonctionnalisée pour l'immobilisation de biomolécules et servant de produits de départ pour la fabrication de microarrays, dans le cadre duquel la surface d'un support est revêtue d'un initiateur et cette surface revêtue mise en contact avec une solution contenant au moins un premier groupe de monomères polymérisables, les monomères contenant des sites de liaison pouvant lier des biomolécules (molécules sondes) et les conditions dans lesquelles la solution de monomère est mise en contact avec le support activé étant choisies de façon telle que les monomère, par l'intermédiaire de l'initiateur, se lient au support et, partant de là, polymérisent pour former des chaînes polymères fonctionnelles conformées de façon à produire une structure de chaînes polymères fonctionnelles voisines fixée à la surface du support, les supports étant de conformation plane et présentant, dans une région de leur surface destinée à la fonctionnalisation, des concavités en forme de cône ou de pyramide dont la pointe est orientée vers l'intérieur du support.

2. Procédé selon la revendication 1, **caractérisé en ce qu'**il met en oeuvre des substances photoactivables, notamment de la benzophénone en guise d'initiateur.

3. Procédé selon la revendication 1 ou 2, **caractérisé en ce que** les supports sont en verre ou en matière plastique.

4. Procédé selon la revendication 3, **caractérisé en ce que** les supports en matière plastique sont en polystyrène, en polycarbonate, en chlorure de polyvinyle ou en polypropylène.

5. Procédé selon la revendication 4, **caractérisé en ce que** les concavités sont de formes et de dimensions correspondantes et pratiquées dans la surface du support à des intervalles correspondants.

6. Procédé selon l'une des revendications 1 à 5, **caractérisé en ce que** les supports en verre mis en oeuvre par le procédé sont, en une première étape, silanisés.

7. Procédé selon l'une des revendications 1 à 6, **caractérisé en ce qu'**un second groupe au moins de monomères est copolymérisé avec le premier groupe sur le support, les monomères du second groupe ne présentant, pour l'essentiel, pas de sites de liaison pour des biomolécules.

8. Procédé selon la revendication 7, **caractérisé en ce que** les monomères du second groupe sont choisis et leur concentration déterminées de façon à obtenir une hydrophilie ou hydrophobie voulue pour les chaînes polymères fonctionnelles copolymérisées.

9. Procédé selon la revendication 8, **caractérisé en ce que** le choix et la concentration des monomères du second groupe sont tels que l'on obtient des chaînes polymères fonctionnelles copolymérisées qui accueillent une goutte déposée lors du spotting en en maintenant la structure tridimensionnelle.

10. Procédé selon l'une des revendications 1 à 9, **caractérisé en ce que** les monomères du premier et du second groupe sont choisis parmi les composés suivants : acides acryliques, acides méthacryliques, dérivés de l'acide acrylique ou méthacrylique, composés vinyliques ou allyliques.

11. Procédé selon la revendication 10, **caractérisé en ce que** les monomères du premier groupe sont choisis parmi les composés suivants : acides acryliques, méthacrylates de glycidyle, méthacrylates d'aminoalkyle.

12. Procédé selon la revendication 11, **caractérisé en ce que** les monomères du second groupe sont choisis parmi les composés suivants : polyéthylèneglycols, méthacrylates ou méthacrylamides d'hydroxyméthyle.

13. Procédé selon l'une des revendications 1 à 12, **caractérisé en ce que** les sites de liaison que comportent les monomères du premiers groupes sont des groupes fonctionnels, notamment des groupes COOH-, SH-, NH₂-, époxyde ou thiol.

14. Procédé de fabrication de microarrays dans le cadre duquel des molécules sondes dissoutes dans un solvant sont déposées goutte à goutte dans des régions définies sur un support à surface modifiée réalisé conformément aux revendications 1 à 13.

15. Procédé de fabrication de microarrays selon la revendication 14 dans le cadre duquel les supports à surface modifiée sont revêtus de molécules sondes par le procédé du spotting.

16. Procédé selon la revendication 14 ou 15, **caractérisé en ce qu'**il met en oeuvre des oligonucléotides ou des anticorps en guise de molécules sondes.

17. Procédé de fabrication de microarrays selon l'une des revendications 14 à 16, **caractérisé en ce que** les microarrays sont façonnés pour servir de puces destinées à l'analyse d'acides nucléiques.

18. Support à surface modifiée pouvant être fabriqué conformément aux revendications 1 à 13.

19. Support destiné à la technique de liaison sous forme de tableau pouvant être fabriqué conformément aux revendications 14 à 17.
